# FASCICULE DE BREVET EUROPEEN

(11) **EP 0 937 085 B1**
(45) Date de publication et mention de la délivrance du brevet: **22.01.2003**
(21) Numéro de dépôt: 97911285.1
(22) Date de dépôt: 17.10.1997
(51) Int. Cl.: C07F 3/02

(54) **LE (-)HYDROXYCITRATE DE MAGNESIUM, PROCEDE DE PREPARATION, APPLICATIONS, ET COMPOSITIONS NOTAMMENT PHARMACEUTIQUES LE RENFERMANT**
MAGNESIUM(-)HYDROXYCITRAT, VERFAHREN ZUR HERSTELLUNG, ANWENDUNG UND PHARMAZEUTISCHE ZUSAMMENSETZUNGEN, DIE SIE ENTHALTEN
MAGNESIUM (-)HYDROXYCITRATE, METHOD OF PREPARATION, APPLICATIONS, AND COMPOSITIONS IN PARTICULAR PHARMACEUTICAL CONTAINING SAME

(30) Priorité: 22.10.1996 FR 9613094
(43) Date de publication de la demande: 25.08.1999
(73) Titulaire: Shrivastava, Ravi, 63118 Cebazat (FR); Lambropoulos, Patrick, 13007 Marseille (FR)
(72) Inventeur: Shrivastava, Ravi, 63118 Cebazat (FR); Lambropoulos, Patrick, 13007 Marseille (FR)
(74) Mandataire: Rinuy, Santarelli
(86) Numéro de dépôt international: FR9701860
(87) Numéro de publication internationale: WO98017671

(56) Documents cités:
- FR-A- 2 716 374
- FR-A- 2 733 418
- CHEMICAL ABSTRACTS, vol. 124, no. 8, 1996 Columbus, Ohio, US; abstract no. 97375r, page 743; colonne 1; XP002034986 & R.P. SINGH ET AL: BIOL. MEM., vol. 21, no. 1, 1995, pages 27-33,

## Description

La présente invention concerne un nouveau composé du (-)hydroxycitrate, son procédé de préparation et les compositions le renfermant.

Le (-)hydroxycitrate (acide (-)hydroxycitrique ou encore acide Garcinia) est le principe actif obtenu de l'extrait de Garcinia indica ou de Garcinia cambogia, arbres du Sud-Est asiatique et plus particulièrement du sud de l'Inde.

Ces deux espèces de plantes renferment directement un stéréo-isomère (-) de l'hydroxycitrate. L'extrait des fruits de ces deux plantes est largement utilisé dans la médecine traditionnelle indienne pour le traitement de diverses maladies.

Un mélange de (-)hydroxycitrate et de chrome est commercialisé aux Etats-Unis en tant que produit diététique pour lutter contre la surcharge pondérale et l'obésité, sous le nom de Citrin®.

Des activités pharmacologiques connues et des procédés de préparation du (-)hydroxycitrate à partir de fruits sont déjà décrits dans la littérature.

Ce produit est en outre commercialisé par différents fabricants tels que la Société FLUKA.

Majeed M. et al dans : "CITRIN® : a revolutionnary herbal approach to weight management" New Editions, Publishing 1675, Rollins Road, Burlingame, CA 94010 Pages 1-69, 1994, décrit un certain nombre d'études qui montrent que le (-)hydroxycitrate réduit le poids corporel par réduction de la synthèse de matière grasse.

EP-A- 0739 629 décrit des associations synergiques comprenant de l'acide (-) hydroxycitrique encore appelé acide Garcinia et soit un métal ionisé ou non, soit une vitamine, ainsi que leurs utilisations pour la préparation de médicaments destinés au traitement d'une maladie cardio-vasculaire, en tant que produit diététique, nutritionnel ou cosmétique. Il est évoqué que le métal ionisé peut être combiné à l'ion (-) hydroxycitrate et le (-) hydroxycitrate de magnésium est spécifiquement cité.

FR-A-2 716 374 décrit des extraits de plantes riches en acide hydroxycitrique et leur utilisation notamment pour réguler la lipogénèse et pour le renouvellement cellulaire cutané.

De nombreuses personnes dans le monde, par exemple environ 4 millions de personnes en France, souffrent d'hypercholestérolémie. Lorsque les niveaux de cholestérol circulant excédent les niveaux normaux, en raison de la péroxydation des lipides, le cholestérol et les esters de cholestérol sont capables de s'accumuler dans les cellules des muscles lisses artériels, provoquant leur prolifération, l'accumulation de lipides sur la paroi artérielle (athérome), pouvant engendrer la formation de thrombus, suivie par le blocage d'artères coronaires ou cérébrales.

Les traitements existants (fibrates, inhibiteurs de HMG coenzyme-A réductase, etc ...) s'appliquent à réduire les niveaux de cholestérol circulant mais ne jouent aucun rôle de protection dans les effets pathologiques du cholestérol sur la paroi artérielle, que ce soit en réduisant la prolifération ou en inhibant l'accumulation du cholestérol dans les cellules des muscles lisses artériels.

En outre, en raison de leurs effets secondaires, tels que l'insuffisance hépatique ou rénale, ces médicaments sont utilisés uniquement lorsque les niveaux de cholestérol circulant excèdent largement les limites physiologiques.

Il serait donc souhaitable de trouver un produit doué des propriétés suivantes :
- capable de réduire la synthèse de cholestérol,
- capable d'inhiber l'accumulation des lipides dans les cellules des muscles lisses vasculaires,
- capable d'aider à l'élimination des lipides accumulés dans les cellules des muscles lisses vasculaires,
- capable de réduire la prolifération cellulaire due à la réduction des lipides intracellulaires,
- pourvu d'une marge thérapeutique suffisante, et
- dénué d'effets secondaires ou d'effets toxiques à de hautes doses.

C'est pourquoi la présente invention a pour objet un nouveau composé du (-)hydroxycitrate, à savoir le (-)hydroxycitrate de magnésium, notamment le (-)hydroxycitrate de magnésium cristallisé, particulièrement substantiellement pur.

La présente invention a également pour objet un procédé de préparation du (-)hydroxycitrate de magnésium, caractérisé en ce que l'on fait réagir un extrait de de Garcinia cambogia avec un alcool aliphatique pour obtenir un précipité que l'on soumet à l'action d'un agent fixateur des tanins, élimine les solides et récupère le surnageant que l'on soumet à une chromatographie en batch sur résine échangeuse d'anions, laisse en contact sous agitation, élimine le sumageant, procède à l'élution du (-)hydroxycitrate de magnésium, et sèche l'éluat pour obtenir le (-)hydroxycitrate de magnésium attendu.

Dans des conditions préférentielles de mise en oeuvre du procédé ci-dessus décrit,
- l'alcool aliphatique est un alcool en C₁-C₆ de préférence le propanol, l'isopropanol ou l'éthanol
- l'agent fixateur des tanins est un dérivé de la Polypyrrolidone, de préférence la Polyvinyl Polypyrrolidone,
- l'élimination des solides est réalisée par centrifugation,
- la résine échangeuse d'anions est de préférence une résine DEAE SEPHADEX A 50.
- le séchage de l'éluat est effectué par lyophilisation.

Le (-)hydroxycitrate de magnésium possède de très intéressantes propriétés pharmacologiques. Il est doué notamment de remarquables propriétés hypolipémiantes, anticholestérol, antiathéromateuses et antioxydantes notamment vis à vis des radicaux libres. Il est en particulier capable de réduire la synthèse de cholestérol, d'inhiber l'accumulation des lipides dans les cellules des muscles lisses vasculaires, d'aider à l'élimination des lipides accumulés dans les cellules des muscles lisses vasculaires, de réduire la prolifération cellulaire due à la réduction des lipides intracellulaires, et en conséquence, diminuer le dépôt de graisses sur l'endothélium vasculaire. Il est en outre doué de propriétés antistress, antifatigue, et régénérantes cellulaires. Il a aussi été trouvé doué de propriétés antihypertensives.

Ces propriétés sont illustrées ci-après dans la partie expérimentale. Elles justifient l'utilisation du (-)hydroxycltrate de magnésium à titre de médicament.

Les médicaments selon la présente invention trouvent leur emploi par exemple dans le traitement tant curatif que préventif des pathologies induites par l'accumulation locale des lipides ou par l'augmentation du taux de cholestérol circulant qui induit des maladies telles que la sténose vasculaire, les stries lipidiques, la formation d'athérome, la thrombose et les maladies vasculaires affectant tant la macro- que la microcirculation ainsi que l'hypercholestérolémie.

Ils trouvent aussi leur emploi dans les troubles de la sénescence liés aux effets des oxydants tels que les radicaux libres.

Ils trouvent également leur emploi dans le traitement tant curatif que préventif de l'hypertension artérielle.

La dose usuelle, variable selon le sujet traité et l'affection en cause, peut être, par exemple, de 100 à 1000 mg par jour par voie orale chez l'homme de (-)hydroxycitrate de magnésium, pendant au moins 20 jours.

L'invention a aussi pour objet les compositions pharmaceutiques qui renferment au moins le (-)hydroxycitrate de magnésium, à titre de principe actif.

Dans ces compositions, le principe actif est avantageusement présent à des doses physiologiquement efficaces ; les compositions précitées renferment notamment une dose antihypercholestérolémiante ou antihypertensive efficace d'au moins un principe actif ci-dessus.

A titre de médicaments, le (-)hydroxycitrate de magnésium peut être incorporé dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés simples ou dragéifiés, les gélules, les granulés, les caramels, les suppositoires, les préparations injectables les crèmes, lotions ou gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La demanderesse a en outre découvert que le (-)hydroxycitrate de magnésium était capable de potentialiser les effets de minéraux, notamment des métaux impliqués dans les réactions enzymatiques physiologiques, tout particulièrement le zinc, le cuivre, le manganèse, le sélénium et le silicium, et était aussi capable de potentialiser les effets de vitamines, particulièrement les vitamines A, C et E.

Mais, si les produits précités, minéraux et vitamines, ont montré de légers effets de protection vis à vis de l'hypercholestérolémie, ces effets ne sont pas d'un niveau suffisant pour envisager une utilisation thérapeutique. Cependant, potentialisés par le (-)hydroxycitrate de magnésium, ils ont des effets tout à fait surprenants.

C'est pourquoi la présente invention a encore pour objet une association synergique, caractérisée en ce qu'elle comprend du (-)hydroxycitrate de magnésium et au moins un métal, sous forme ionisée ou non, de préférence sous forme de cation, choisi dans le groupe constitué par les métaux suivants : magnésium, cuivre, cobalt, zinc, nickel, sélénium, silicium, manganèse, lithium et fer, ou du (-)hydroxycitrate de magnésium et au moins une vitamine, notamment une vitamine antioxydante.

Des associations synergiques préférées selon l'invention comprennent du (-)hydroxycitrate de magnésium et en outre au moins deux métaux, notamment deux cations choisis dans le groupe précité et/ou au moins deux vitamines.

Dans des conditions préférentielles, les constituants de l'association synergique selon l'invention sont présents dans les proportions relatives pondérales suivantes : pour une partie de (-)hydroxycitrate de magnésium, 0,1 à 2 parties d'un sel minéral ou d'un oxyde d'un métal ci-dessus et/ou 0,1 à 1 partie de vitamine(s), sachant que dans un sel minéral ou un oxyde, le métal peut représenter pondéralement de 2 à 50 % en poids, de préférence de 5 à 40 %, particulièrement 10 à 35 %, par rapport en poids du composé considéré.

Parmi les métaux précités, on préfère le sélénium et le silicium, de préférence sous forme de cations.

Les anions avec lesquels peuvent être combinés les cations ci-dessus sont les anions pharmaceutiquement acceptables comme par exemple ceux dérivés des acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques ou carboxyliques, ou l'acide gluconique.

A titre de vitamines, on retient de préférence les vitamines A, C et E et tout particulièrement les vitamines A et E.

Les associations selon la présente invention se présentent de préférence en doses unitaires comprenant chacune au moins 50 mg de (-)hydroxycitrate de magnésium, de préférence au moins 100 mg de (-)hydroxycitrate de magnésium, et tout particulièrement environ 250 mg de (-)hydroxycitrate de magnésium. Avantageusement, une dose unitaire comprend au plus 1 g de (-)hydroxycitrate de magnésium. En particulier, une forme unitaire se présente sous un volume inférieur à 20 cm³, notamment inférieur à 10 cm³, tout particulièrement inférieur à 1 cm³.

Les associations objet de la présente invention possèdent les mêmes très intéressantes propriétés pharmacologiques que celles du (-)hydroxycitrate de magnésium.

Ces propriétés sont illustrées plus loin dans la partie expérimentale. Elles justifient donc l'utilisation des associations ci-dessus décrites à titre de médicament.

La dose usuelle, variable selon le sujet traité et l'affection en cause, peut être, par exemple, de 100 à 1000 mg par jour par voie orale chez l'homme de (-)hydroxycitrate de magnésium, pendant au moins 20 jours, associé à 2 à 40 mg de silice colloïdale et à 100 à 1500 mg de gluconate de sélénium ou de magnésium.

La présente invention a également pour objet un procédé de préparation d'une association telle que définie ci-dessus caractérisée en ce que l'on mélange le (-)hydroxycitrate de magnésium avec au moins un métal sous forme ionisée ou non ou avec au moins une vitamine antioxydante précitée.

La présente invention a également pour objet une association renfermant du (-)hydroxycitrate de magnésium ou une association de (-)hydroxycitrate de magnésium et d'au moins un métal sous forme ionisée ou non choisi dans le groupe constitué par les métaux suivants : magnésium, cuivre, cobalt, zinc, nickel, sélénium, silicium, manganèse, lithium et fer, ou du (-)hydroxycitrate et d'au moins une vitamine, notamment antioxydante, à titre de médicament, c'est à dire le (-)hydroxycitrate de magnésium ou une association de composés ci-dessus pour son utilisation dans une méthode de traitement thérapeutique, curatif ou préventif du corps humain ou animal.

A titre de traitement thérapeutique, on peut citer notamment les applications et indications connues de ces métaux ou vitamines ; en effet, leurs effets sont potentialisés par le (-)hydroxycitrate de magnésium.

La présente invention a encore pour objet l'application du (-)hydroxycitrate de magnésium ou d'une association renfermant du (-)hydroxycitrate de magnésium et d'au moins un métal sous forme ionisée ou non choisi dans le groupe constitué par les métaux suivants : magnésium, cuivre, cobalt, zinc, nickel, sélénium, silicium, manganèse, lithium et fer, ou du (-)hydroxycitrate de magnésium et une vitamine notamment antioxydante à titre de produit diététique, complément nutritionnel, adjuvant nutritionnel ou encore à titre de produit cosmétique.

L'invention a donc aussi pour objet des compositions diététiques, nutritionnelles ou cosmétiques renfermant le (-)hydroxycitrate de magnésium ou une association ci-dessus.

La présente invention a en outre pour objet le (-)hydroxycitrate de magnésium ou une association synergique de (-)hydroxycitrate de magnésium et d'au moins un métal choisi dans le groupe constitué par les métaux suivants : magnésium, cuivre, cobalt, zinc, nickel, sélénium, silicium, manganèse, lithium et fer, ou du (-)hydroxycitrate de magnésium et/ou d'au moins une vitamine, notamment antioxydante, pour la préparation d'un médicament destiné au traitement d'une maladie cardio-vasculaire, notamment destiné au traitement d'une pathologie localisée ou généralisée induite par le cholestérol ou de l'hypertension.

Dans des conditions préférentielles de réalisation, le médicament destiné au traitement d'une maladie cardio-vasculaire ci-dessus est un médicament à propriétés antioxydantes des lipides.

Dans des conditions tout particulièrement préférées, le médicament ci-dessus est destiné au traitement de l'hypercholestérolémie et des pathoiogies induites par celle-ci ou au traitement de l'hypertension artérielle.

Les exemples qui suivent illustrent la présente demande.

### PARTIE EXPERIMENTALE

### Exemple 1 : Préparation du (-)hydroxycitrate de magnésium

On fait réagir 1 Kg d'un extrait à 30% de Garcinia cambogia (Fournisseur GREENTECH St Beauzire-FRANCE) titrant 60g/l d'acide hydroxycitrique avec 2 Litres d'éthanol pour obtenir un précipité que l'on soumet à l'action de 50 g de Polyvinyl Polypyrrolidone, porte 30 minutes sous agitation, élimine les solides par centrifugation 15 minutes à 6000 g et récupère le sumageant. On soumet ce dernier à une chromatographie en batch sur 200g de résine échangeuse d'anions DEAE SEPHADEX A 50 équilibrée à pH 3, NaCl 0.1M (1 g de résine pour 1,5 mole d'acide), laisse agiter pendant 16 heures, décante, élimine le surnageant, élue avec 1000 ml d'une solution 0,5 M de chlorure de magnésium, laisse agiter pendant 4 heures, récupère l'éluat, sèche et obtient 70 g du produit attendu.

### EXEMPLE 2

On a préparé des comprimés répondant à la formule

| | |
|---|---|
| (-)hydroxycitrate de magnésium (principe actif) | 300 mg |
| Gluconate de magnésium | 360 mg |
| Silice colloïdale | 7,5 mg |
| excipient q.s. pour un comprimé terminé à | 1 000 mg |
| (détail de l'excipient: lactose, amidon, talc, stéarate de magnésium). | |

### EXEMPLE 3

On a préparé des comprimés sécables répondant à la formule :

| | |
|---|---|
| (-)hydroxycitrate de magnésium | 150 mg |
| Selenium | 5 mg |
| Vitamine E | 75 mg |
| excipient q.s. pour un comprimé terminé à | 250 mg |
| (détail de l'excipient : lactose, amidon, talc, stéarate de magnésium). | |

### EXEMPLE 4

On a préparé des comprimés sécables répondant à la formule :

| | |
|---|---|
| (-)hydroxycitrate de magnésium | 375 mg |
| silice colloïdale | 7,5 mg |
| sélénium | 40 mg |
| excipient q.s. pour un comprimé sécable terminé à | 1000 mg |

### EXEMPLE 5

On a préparé des ampoules buvables répondant à la formule :

| | |
|---|---|
| (-)hydroxycitrate de magnésium | 300 mg |
| gluconate de magnésium | 100 mg |
| sélénium | 40 µg |
| excipient q.s. pour une ampoule terminée à | 10 ml |
| (détail de l'excipient : eau distillée) | |

### EXEMPLE 6

On a préparé des sachets pour suspension buvable répondant à la formule :

| | |
|---|---|
| (-)hydroxycitrate de magnésium | 500 mg |
| gluconate de magnésium | 100 mg |
| excipient q.s. pour un sachet terminé à | 5 g |
| (détail de l'excipient : lactose) | |

### EXEMPLE 7

On a préparé des sachets pour suspension buvable répondant à la formule :

| | |
|---|---|
| (-)hydroxycitrate de magnésium | 250 mg |
| gluconate de magnésium | 150 mg |
| excipient q.s. pour un sachet terminé à | 5 g |
| (détail de l'excipient : lactose) | |

### Etude Pharmacologique

### 1) Etude de mortalité de cellules humaines épithéliales et de cellules de muscle lisse artériel du rat.

On a testé les effets antioxydants du (-)hydroxycitrate et de différents minéraux selon le protocole décrit par Michiels et al : "A new experimental model to study oxygen toxicity", Arch. int. Physiol. Biochem. 94 (5), S13-S18, 1986, en utilisant des hépatocytes de rat, ainsi que des cellules épithéliales humaines et des cellules de muscle lisse artériel de rat.

On a également testé les effets inhibiteurs de ces composés sur la prolifération du muscle lisse artériel et sur l'accumulation de lipides intracellulaires, en utilisant la méthode décrite par Shrivastava et al dans Meth. Find. Exp. Clin. Pharmacol. 15 (6), pages 345-350, 1993.

Les divers produits ont été respectivement testés aux concentrations suivantes :
- (-)hydroxycitrate 0,1 mM
- silicium 0,036 mM
- magnésium 0,1 mM
dans le milieu de culture.

Les résultats obtenus sont les suivants :

| REDUCTION EN POURCENTAGES | | | |
|---|---|---|---|
| Produit testé | mort cellulaire | Accumulation des lipides | prolifération des cellules |
| (-)hydroxycitrate | 0 | 16 (±3) | 6 (±4) |
| Magnésium | 6 (±3) | 20 (±9) | 2 (±2) |
| Silicium | 28 (±7) | 32 (±5) | 28 (±6) |
| (-)hydroxycitrate de magnésium | 70 (±8) | 81 (±13) | 48 (±8) |

On observe d'une part que le (-)hydroxycitrate seul n'a aucun effet de protection sur la mortalité cellulaire. Le magnésium seul diminue très faiblement cette mortalité, tandis que le (-)hydroxycitrate de magnésium a un effet plus protecteur.

Le (-)hydroxycitrate de magnésium diminue fortement la mortalité cellulaire (70 ± 8%), prouvant l'effet protecteur de ce produit contre les dommages oxydatifs.

En ce qui concerne l'accumulation des lipides et la prolifération cellulaire, on observe une augmentation très importante des effets du (-)hydroxycitrate de magnésium par rapport au (-)hydroxycitrate ou au magnésium.

Ces résultats sont tout à fait inattendus

### 2) Etude d'effet in vivo sur le taux de cholestérol et les dépôts lipidiques chez les lapins hypercholestérolémiques.

Deux lots de 20 lapins mâles (Elevage scientifique des Dombes : 2,5 - 3 kg) ont reçu une nourriture riche en cholestérol (1 %) pendant une période de 8 semaines. Le premier lot n'a reçu aucun traitement et a donc servi de témoin tandis que le deuxième lot a reçu 750 mg/kg de (-)hydroxycitrate de magnésium par voie orale. Les analyses lipidiques après 4 semaines de traitement ont montré une diminution des LDL et une augmentation des HDL chez les lapins qui ont reçu du (-)hydroxycitrate de magnésium.

| | HDL en g/l | LDL en g/l | Cholestérol total en g/l | Rapport Cholestérol total/HDL |
|---|---|---|---|---|
| Témoins + | 0,224 | 5,17 | 5,55 | 26,78 |
| (-)hydroxycitrate de magnésium | 0,246 + 8,5% | 4,00 - 22,4% | 4,47 - 19,5% | 19,22 - 28,2% |

Les analyses effectuées sur la paroi de l'aorte après 4 semaines (10 lapins) et après 8 semaines (10 lapins) ont montré une diminution de l'indice de dépôts lipidiques chez les lapins qui ont reçu du (-)hydroxycitrate de magnésium.

| | Témoins + | (-)hydroxycitrate de magnésium | Variation % |
|---|---|---|---|
| Arche aortique | | | |
| Semaine 4 | 9,5 | 3,5 | |
| Semaine 8 | 14,0 | 1,5 | |
| Arche thoracique | | | |
| Semaine 4 | 0,0 | 0,0 | |
| Semaine 8 | 2,0 | 0,0 | |
| Total | 25,5 | 5,0 | -80,4% |

### 3) Etude des effets antiathérosclérotiques chez le lapin hypercholestérolémique.

Deux lots de 15 lapins mâles (Elevage scientifique des Dombes : 2,5 - 3 kg) ont reçu une nourriture riche en cholestérol : 0,2 % pendant la période J+ 8 à J+ 37, puis 1 % jusqu'à J+ 69, date de sacrifice des animaux.

Le premier lot n'a reçu aucun traitement et a donc servi de témoin tandis que le deuxième lot a reçu 500 mglkg de (-)hydroxycitrate de magnésium par voie orale. Les analyses après la fin du traitement ont montré que l'administration de l'hydroxycitrate de magnésium par voie orale à la dose de 500 mg/kg/jour chez les lapins hypercholestérolémiques pendant 62 jours exerce les effets suivants :
- Absence de mortalité.
- Bonne tolérance
- Absence d'effet notable sur la consommation alimentaire ou le poids corporel par rapport aux témoins.
- Absence de modification dans les paramètres hématologiques.
- Diminution des enzymes hépatiques indiquant un effet hépato protecteur dans le lot traité.
- Réduction du taux de cholestérol total (-13,75%), des LDL (16,36%), et du ratio entre le cholestérol total et les HDL (-25,72%) avec une augmentation du taux des HDL (+12,5%) chez les lapins traités par rapport aux témoins.
- Baisse des poids du coeur, du foie, de la rate (-20,3%) et des glandes surrénales (-21,6%) dans le lot traité indiquant un effet protecteur contre l'hypercholestérolémie sur ces organes et en conséquence une diminution du stress.
- Diminution de 46,3% de dépôts lipidiques sur la paroi vasculaire aortique dans le lot traité par rapport au lot témoin.

Ces résultats montrent que l'hydroxycitrate de magnésium exerce un effet hypocholestérolémiant et anti athérosclérotique à des doses non cytotoxiques chez le lapin hypercholestérolémique.

### 4) Etude d'activité antihypertensive chez le rat hypertendu

On a testé le (-)hydroxycitrate de magnésium sur 12 rats hypertendus. Un lot de 12 autres rats hypertendus a été utilisé comme lot témoin. Une diminution moyenne de la pression artérielle de 17 % ± 3 % par rapport aux témoins (non traités) a été constatée après une semaine de traitement à raison de 500 mg par kilogramme et par jour par voie orale.

### 5) Toxicité

La toxicité du (-)hydroxycitrate de magnésium est supérieure à 2 g/kg par voie intrapéritonéale ou à 7 g/kg per os chez le rat. Celle des métaux, notamment sous forme de sels ou d'oxydes minéraux (fréquemment administrés en tant qu'oligo-éléments) est bien connue.

## Revendications (Revendications pour l'(les) Etat(s) contractant(s) suivant(s): AT, DK, FI, GR, IE, LU, MC, PT, SE)

1. Le (-)hydroxycitrate de magnésium.

2. Procédé de préparation du (-)hydroxycitrate de magnésium, **caractérisé en ce que** l'on fait réagir un extrait de Garcinia cambogia avec un alcool aliphatique pour obtenir un précipité que l'on soumet à l'action d'un agent fixateur des tanins, élimine les solides et récupère le surnageant que l'on soumet à une chromatographie en batch sur résine échangeuse d'anions, laisse en contact sous agitation, élimine le surnageant, procède à l'élution du (-)hydroxycitrate de magnésium, et sèche l'éluat pour obtenir le (-)hydroxycitrate de magnésium attendu.

3. Le (-)hydroxycitrate de magnésium pour son utilisation dans une méthode de traitement thérapeutique, curatif ou préventif, du corps humain ou animal.

4. Utilisation du (-)hydroxycitrate de magnésium pour la préparation d'un médicament destiné au traitement d'une maladie cardio-vasculaire.

5. Une composition pharmaceutique, **caractérisée en ce qu'**elle renferme du (-)hydroxycitrate de magnésium et un excipient pharmaceutiquement acceptable.

6. Une composition, **caractérisée en ce qu'**elle comprend du (-)hydroxycitrate de magnésium et au moins un métal sous forme ionisée ou non, choisi dans le groupe constitué par les métaux suivants : magnésium, cuivre, cobalt, zinc, nickel, sélénium, silicium, manganèse, lithium et fer, ou du (-)hydroxycitrate de magnésium et au moins une vitamine.

7. Une composition synergique selon la revendication 6, **caractérisée en ce qu'**elle comprend au moins deux métaux choisis dans le groupe constitué par les métaux suivants: magnésium, cuivre, cobalt, zinc, nickel, sélénium, silicium, manganèse, lithium et fer.

8. Une composition selon la revendication 6 ou 7, sous forme d'une dose unitaire comprenant au moins 50 mg de (-)hydroxycitrate de magnésium.

9. Une composition selon l'une des revendications 6 à 8, **caractérisée en ce qu'**elle renferme pondéralement, pour une partie de (-)hydroxycitrate de magnésium, 0,1 à 2 parties d'un sel minéral ou d'un oxyde d'un métal ou pour une partie de (-)hydroxycitrate de magnésium, 0,1 à 1 partie d'au moins une vitamine.

10. Utilisation du (-)hydroxycitrate de magnésium ou d'une composition telle que définie à l'une des revendications 6 à 9, à titre de produit diététique, ou nutritionnel.

11. Utilisation du (-)hydroxycitrate de magnésium ou d'une composition telle que définie à l'une des revendications 6 à 9, à titre de produit cosmétique.

## Revendications (Revendications pour l'(les) Etat(s) contractant(s) suivant(s): CH, DE, ES, FR, GB, IT, BE, NL)

1. Procédé de préparation du (-)hydroxycitrate de magnésium, **caractérisé en ce que** l'on fait réagir un extrait de Garcinia cambogia avec un alcool aliphatique pour obtenir un précipité que l'on soumet à l'action d'un agent fixateur des tanins, élimine les solides et récupère le surnageant que l'on soumet à une chromatographie en batch sur résine échangeuse d'anions, laisse en contact sous agitation, élimine le sumageant, procède à l'élution du (-)hydroxycitrate de magnésium, et sèche réluat pour obtenir le (-)hydroxycitrate de magnésium attendu.

## Patentansprüche (Patentansprüche für folgende(n) Vertragsstaat(en): AT, DK, FI, GR, IE, LU, MC, PT, SE:)

1. (-)-Magnesiumhydroxycitrat

2. Verfahren zur Herstellung von (-)-Magnesiumhydroxycitrat, **dadurch gekennzeichnet, dass** ein Extrakt von Garcinia cambogia mit einem aliphatischen Alkohol zur Umsetzung gebracht wird, um einen Niederschlag zu erhalten, welcher der Wirkung eines Tannin-Fixiermittels unterzogen wird, die Feststoffe entfernt und der Überstand gewonnen wird, der einer diskontinuierlichen Anionenaustausch-Chromatographie unterzogen wird, unter Rühren in Kontakt stehen gelassen wird, der Überstand entfernt wird, die Verdünnung des (-)-Magnesiumhydroxycitrats vorgenommen und das Eluat getrocknet wird, um das erwartete (-)-Magnesiumhydroxycitrat zu erhalten.

3. (-)-Magnesiumhydroxycitrat zur Verwendung in einem Verfahren zur therapeutischen, kurativen oder präventiven Behandlung des menschlichen oder tierischen Körpers.

4. Verwendung von (-)-Magnesiumhydroxycitrat zur Herstellung eines Arzneimittels zur Behandlung einer kardiovaskulären Erkrankung.

5. Pharmazeutische Zusammensetzung, **dadurch gekennzeichnet, dass** sie (-)-Magnesiumhydroxycitrat und einen pharmazeutisch annehmbaren Exzipienten enthält.

6. Zusammensetzung, **dadurch gekennzeichnet, dass** sie (-)-Magnesiumhydroxycitrat und mindestens ein Metall in gegebenenfalls ionisierter Form, ausgewählt aus der Gruppe bestehend aus den Metallen Magnesium, Kupfer, Kobalt, Zink, Nickel, Selen, Silizium, Mangan, Lithium und Eisen, oder(-)-Magnesiumhydroxycitrat und mindestens ein Vitamin umfasst.

7. Synergetische Zusammensetzung nach Anspruch 6, **dadurch gekennzeichnet, dass** sie mindestens zwei Metalle ausgewählt aus der Gruppe bestehend aus den Metallen Magnesium, Kupfer, Kobalt, Zink, Nickel, Selen, Silizium, Mangan, Lithium und Eisen umfasst.

8. Zusammensetzung nach Anspruch 6 oder 7 in Dosiseinheitsform umfassend mindestens 50 mg (-)-Magnesiumhydroxycitrat.

9. Zusammensetzung nach einem der Ansprüche 6 bis 8, **dadurch gekennzeichnet, dass** sie gewichtsmäßig pro Teil (-)-Magnesiumhydroxycitrat 0,1 bis 2 Teile eines Mineralsalzes oder eines Metalloxids oder pro Teil (-)-Magnesiumhydroxycitrat 0,1 bis 1 Teil mindestens eines Vitamins enthält.

10. Verwendung von (-)-Magnesiumhydroxycitrat oder einer Zusammensetzung nach einem der Ansprüche 6 bis 9 als diätetisches Produkt oder als Nahrungsmittelzusatz.

11. Verwendung von (-)-Magnesiumhydroxycitrat oder einer Zusammensetzung nach einem der Ansprüche 6 bis 9 als kosmetisches Produkt.

## Patentansprüche (Patentansprüche für folgende(n) Vertragsstaat(en): CH, DE, ES, FR, GB, IT, BE, NL:)

1. Verfahren zur Herstellung von (-)-Magnesiumhydroxycitrat, **dadurch gekennzeichnet, dass** ein Extrakt von Garcinia cambogia mit einem aliphatischen Alkohol zur Umsetzung gebracht wird, um einen Niederschlag zu erhalten, welcher der Wirkung eines Tannin-Fixiermittels unterzogen wird, die Feststoffe entfernt und der Überstand gewonnen wird, der einer diskontinuierlichen Anionenaustausch-Chromatographie unterzogen wird, unter Rühren in Kontakt stehen gelassen wird, der Überstand entfernt wird, die Verdünnung des (-)-Magnesiumhydroxycitrats vorgenommen und das Eluat getrocknet wird, um das erwartete (-)-Magnesiumhydroxycitrat zu erhalten.

## Claims (Claims for the following Contracting State(s): AT, DK, FI, GR, IE, LU, MC, PT, SE)

1. Magnesium (-)hydroxycitrate

2. Process for the preparation of magnesium (-)hydroxycitrate, **characterized in that** an extract of Garcinia cambogia is reacted with an aliphatic alcohol in order to obtain a precipitate which is subjected to the action of a tannin fixing agent, the solids are eliminated and the supernatant is recovered and subjected to batch chromatography on anion exchange resin, left in contact and under stirring, the supernatant is eliminated, elution of the magnesium (-)hydroxycitrate is carried out, and the eluate is dried in order to obtain the expected magnesium (-)hydroxycitrate.

3. Magnesium (-)hydroxycitrate for its use in a therapeutic, curative or preventative treatment method for the human or animal body.

4. Use of magnesium (-)hydroxycitrate for the preparation of a medicament intended for the treatment of a cardiovascular disease.

5. A pharmaceutical composition, **characterized in that** it comprises magnesium (-)hydroxycitrate and a pharmaceutically acceptable excipient.

6. A composition, **characterized in that** it comprises magnesium (-)hydroxycitrate and at least one metal in ionised or non-ionised form, chosen from the group constituted by the following metals: magnesium, copper, cobalt, zinc, nickel, selenium, silicon, manganese, lithium and iron, or magnesium (-)hydroxycitrate and at least one vitamin.

7. A synergistic composition according to claim 6, **characterized in that** it comprises at least two metals chosen from the group constituted by the following metals: magnesium, copper, cobalt, zinc, nickel, selenium, silicon, manganese, lithium and iron.

8. A composition according to claim 6 or 7, in the form of a unit dose comprising at least 50 mg of magnesium (-)hydroxycitrate.

9. A composition according to one of claims 6 to 8, **characterized in that**, by weight, it contains 0.1 to 2 parts of a mineral salt of a metal oxide per one part of magnesium (-)hydroxycitrate or 0.1 to 1 part of at least one vitamin per one part of magnesium (-)hydroxycitrate.

10. Use of magnesium (-)hydroxycitrate or a composition as defined in one of claims 6 to 9 as a dietetic or nutritional product.

11. Use of magnesium (-)hydroxycitrate or a composition as defined in one of claims 6 to 9 as a cosmetic product.

## Claims (Claims for the following Contracting State(s): CH, DE, ES, FR, GB, IT, BE, NL)

1. Process for the preparation of magnesium (-)hydroxycitrate, **characterized in that** an extract of Garcinia cambogia is reacted with an aliphatic alcohol in order to obtain a precipitate which is subjected to the action of a tannin fixing agent, the solids are eliminated and the supernatant is recovered and subjected to batch chromatography on anion exchange resin, left in contact and under stirring, the supernatant is eliminated, elution of the magnesium (-)hydroxycitrate is carried out, and the eluate is dried in order to obtain the expected magnesium (-)hydroxycitrate.
